(19) Europäisches Patentamt
European Patent Office
Office européen des brevets

(11) EP 3 835 737 B1

(12) **EUROPEAN PATENT SPECIFICATION**

(45) Date of publication and mention of the grant of the patent:
**20.03.2024 Bulletin 2024/12**

(21) Application number: **19214877.3**

(22) Date of filing: **10.12.2019**

(51) International Patent Classification (IPC):
***G01F 23/292*** *(2006.01)* ***G01N 15/05*** *(2006.01)*
***G01N 35/02*** *(2006.01)* *G01N 15/04 (2006.01)*
*G01N 21/17 (2006.01)* *G01N 21/59 (2006.01)*
*G01N 35/10 (2006.01)* *G01N 35/00 (2006.01)*

(52) Cooperative Patent Classification (CPC):
**G01N 15/042; G01F 23/2921; G01N 15/05; G01N 35/1016;** G01N 2015/045; G01N 2035/00752; G01N 2035/1025

(54) **METHOD AND APPARATUS FOR DETERMINING A VERTICAL POSITION OF A HORIZONTALLY EXTENDING INTERFACE BETWEEN A FIRST COMPONENT AND A SECOND COMPONENT**

VERFAHREN UND VORRICHTUNG ZUR BESTIMMUNG EINER VERTIKALEN POSITION EINER HORIZONTAL VERLÄNGERTEN SCHNITTSTELLE ZWISCHEN EINER ERSTEN KOMPONENTE UND EINER ZWEITEN KOMPONENTE

PROCÉDÉ ET APPAREIL PERMETTANT DE DÉTERMINER UNE POSITION VERTICALE D’UNE INTERFACE S’ÉTENDANT HORIZONTALEMENT ENTRE UN PREMIER COMPOSANT ET UN SECOND COMPOSANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.06.2021 Bulletin 2021/24**

(73) Proprietors:
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO RS SE SI SK SM TR**

(72) Inventor: **Rein, Michael**
**70736 Fellbach (DE)**

(74) Representative: **Patentanwälte**
**Ruff, Wilhelm, Beier, Dauster & Partner mbB**
**Kronenstraße 30**
**70174 Stuttgart (DE)**

(56) References cited:
**EP-A1- 3 467 452**
**WO-A1-2019/018313**
**JP-A- 2014 006 094**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 835 737 B1

## Description

The invention relates to a method and an apparatus for determining a vertical position of a horizontally extending interface between a first component and a second component, wherein the first component and the second component are contained in a laboratory sample container in layers that are vertically separated from each other.

EP 2 770 317 A1 discloses an apparatus for determining a vertical position of at least one interface between a first component and at least one second component, the components being comprised as different layers in a sample container or sample tube.

WO 2019/018313 A1 discloses stray light compensating methods and an apparatus for characterizing a specimen. 2D statistical data sets are presented to, and operated on, by a multi-class classifier, which may classify pixels in image data sets as belonging to one of a plurality of class labels, such as a serum or plasma portion, a settled blood portion, a gel separator (if used), air, a tube, a label, and possibly even a cap. From this, the pixels making up the serum and plasma portion as well as the other regions may be identified. The result of the segmentation is consolidated 2D data sets, one data set for each viewpoint where all the pixels therein are classified.

EP 3 467 452 A1 discloses a sample liquid-surface position measurement device and a sample liquid-surface position measurement method.

JP 2014 006094 A discloses a detector for detecting the position of a boundary surface between respective components for a biological sample including a plurality of components.

It is an object of the invention to provide for a method and an apparatus for determining a vertical position of a horizontally extending interface between a first component and a second component having improved characteristics compared to prior art solutions.

By means of the inventive method a vertical position of an interface between a first component and a second component may be determined. The first component and the second component are contained in a laboratory sample container in layers that are vertically separated from each other, if a longitudinal axis of the laboratory sample container is perpendicular to a horizontal plane, i.e. when the laboratory sample container is aligned such that a laboratory sample contained in the laboratory sample container may be processed, e.g. pipetted, etc.

The laboratory sample container may be embodied as a conventional transparent laboratory sample container or as a laboratory sample tube used in automated laboratory instrumentation. The method may be intended to detect horizontal interfaces between different layers of a centrifuged blood sample, such as interfaces between a serum or plasma layer and either a separation medium layer or a cruor (blood cell) layer.

The method comprises the following steps.

First data are generated, wherein the first data depend on the vertical position of the horizontally extending interface. The first data may e.g. be formed as a set of digital data words, wherein for a given vertical position a digital data word of the set of digital data words is assigned, the digital data word depending on properties of the components contained in the laboratory sample container at the given vertical position.

Second data are generated in form of digital picture data of the laboratory sample container containing the first component and the second component. The picture data may e.g. be embodied as raw picture data generated by means of a conventional image sensor or may be embodied as picture data formed by compressing the raw data, e.g. in a jpg-format, etc. The picture data may comprise color information of the laboratory sample container containing the first component and the second component.

A first probability distribution function is computed in response to the first data, wherein the first probability distribution function assigns a probability of the presence/occurrence of the horizontally extending interface to a vertical position.

A second probability distribution function is computed in response to the second data, wherein the second probability distribution function assigns a probability of the presence of the horizontally extending interface to a vertical position. To determine or compute the second probability distribution function conventional image processing methods may be used. By means of the image processing methods e.g. properties of the first component, properties of the second component, properties of the laboratory sample container, and/or properties of labels/barcodes attached to the laboratory sample container may be determined, wherein the second probability distribution function may be computed or determined based on the properties of the first component, the properties of the second component, the properties of the laboratory sample container, and/or the properties of labels/barcodes attached to the laboratory sample container. Finally, the vertical position of the horizontally extending interface is computed depending on the first probability distribution function and on the second probability distribution function.

The first data are generated by sensing a transmittance through the laboratory sample container at different vertical positions. The first data may for this case e.g. be formed as a set of digital data words, wherein for a given vertical position a digital data word of the set of digital data words corresponds to a transmittance at the given vertical position. The transmittance may be sensed for different wavelengths. The first probability distribution function may e.g. be determined based on the disclosure of EP 2 770 317 A1, in particular based on claims 1 and 2, figures 3 and 4 and paragraph [0049].

The vertical position of the horizontally extending interface is determined depending on the first probability distribution function and on the second probability distribution function by combining the first probability distribution function and the second probability distribution function based on Bayes' theorem, Dempster-Shafer theory, and/or Kalman filtering. Reference is made insofar to the relevant technical literature.

According to an embodiment, the method comprises the further steps: providing a test sample container, wherein a first test component and a second test component are contained in the test laboratory sample container in layers that are vertically separated from each other, determining a vertical position of a horizontally extending interface between the first test component and the second test component a number of times, e.g. 1000 to 100000 times, determining a first frequency distribution of the determined vertical positions, and determining the first probability distribution function in response to the first data and in response to the first frequency distribution. The first data may e.g. be used to compute a likely vertical position of the horizontally extending interface between the first component and the second component based on the disclosure of EP 2 770 317 A1. The first probability distribution function is then determined by centering the first frequency distribution at the computed (hypothetical) vertical position.

According to an embodiment, the method comprises the further steps: providing a test sample container having a label attached to the test sample container, determining geometrical properties of the label, e.g. a vertical position of one boundary of the label, a number of times, e.g. 1000 to 100000 times, determining a second frequency distribution of the determined geometrical properties, and determining the second probability distribution function in response to the second data and in response to the second frequency distribution. The second data may e.g. be used to compute vertical positions of vertical boundaries of an attached label, e.g. by means of image processing. Then, the second frequency distribution may be centered at the vertical positions of the vertical boundaries of the attached label. The resulting function may then be used to determine the second probability distribution function.

According to an embodiment, the method comprises the further steps: providing a test sample container having a barcode label attached to the test sample container, determining geometrical properties of the barcode label a number of times, determining a third frequency distribution of the determined geometrical properties, determining the second probability distribution function in response to the second data and in response to the third frequency distribution.

According to an embodiment, the method comprises the further steps: generating the second data comprising color information, wherein the second probability distribution function is determined in response to the color information.

According to an embodiment, the method comprises the further steps: pipetting the first component and/or the second component in response to the determined vertical position of the horizontally extending interface.

The apparatus is adapted for determining a vertical position of a horizontally extending interface between a first component and a second component, wherein the first component and the second component are contained in a laboratory sample container in layers that are vertically separated from each other, the apparatus comprising: a measurement unit being adapted to generate first data, wherein the first data depend on the vertical position of the horizontally extending interface, a digital camera being adapted to generate second data in form of picture data of the laboratory sample container containing the first component and the second component, a processing unit being adapted to determine a first probability distribution function in response to the first data, wherein the first probability distribution function assigns a probability of the presence of the horizontally extending interface to a vertical position, determine a second probability distribution function in response to the second data, wherein the second probability distribution function assigns a probability of the presence of the horizontally extending interface to a vertical position, and determine the vertical position of the horizontally extending interface depending on the first probability distribution function and on the second probability distribution function.

The measurement unit is adapted to generate the first data by sensing a transmittance through the laboratory sample container at different vertical positions. The measurement unit may be embodied as disclosed in EP 2 770 317 A1, in particular as disclosed in fig. 1 of EP 2 770 317 A1.

The apparatus is adapted to perform a method as described above.

The invention will now be described in detail with respect to the attached drawings, wherein

Fig. 1a depicts a test sample container containing a first test component and a second test component in layers that are vertically separated from each other, wherein a horizontally extending interface is formed between the first test component and the second test component,

Fig. 1b depicts a frequency distribution of a number of determined vertical positions of the horizontally extending interface depicted in fig. 1,

Fig. 2a depicts a laboratory sample container containing a first component and a second component in layers that are vertically separated from each other, wherein a horizontally extending interface is formed between the first component and the second component, wherein a label is attached to the laboratory sample container,

Fig. 2b depicts a first probability distribution function, wherein the first probability distribution function assigns a probability of the presence of the horizontally extending interface depicted in fig. 2a to a vertical position, wherein the first probability distribution function is determined in response to first data generated by sensing a transmittance through the laboratory sample container at different vertical positions,

Fig. 2c depicts a second probability distribution function, wherein the second probability distribution function assigns a probability of the presence of the horizontally extending interface depicted in fig. 2a to a vertical position, wherein the second probability distribution function is determined in response to second data in form of picture data of the laboratory sample container,

Fig. 3a depicts a laboratory sample container containing a first component and a second component in layers that are vertically separated from each other, wherein a horizontally extending interface is formed between the first component and the second component, wherein a label and a barcode label are attached to the laboratory sample container,

Fig. 3b depicts a first probability distribution function, wherein the first probability distribution function assigns a probability of the presence of the horizontally extending interface depicted in fig. 3a to a vertical position, wherein the first probability distribution function is determined in response to first data generated by sensing a transmittance through the laboratory sample container at different vertical positions,

Fig. 3c depicts a second probability distribution function, wherein the second probability distribution function assigns a probability of the presence of the horizontally extending interface depicted in fig. 3a to a vertical position, wherein the second probability distribution function is determined in response to second data in form of picture data of the laboratory sample container,

Fig. 4a depicts a laboratory sample container containing a first component and a second component in layers that are vertically separated from each other, wherein a horizontally extending interface is formed between the first component and the second component, wherein a label is attached to the laboratory sample container,

Fig. 4b depicts a first probability distribution function, wherein the first probability distribution function assigns a probability of the presence of the horizontally extending interface depicted in fig. 4a to a vertical position, wherein the first probability distribution function is determined in response to first data generated by sensing a transmittance through the laboratory sample container at different vertical positions,

Fig. 4c depicts a second probability distribution function, wherein the second probability distribution function assigns a probability of the presence of the horizontally extending interface depicted in fig. 4a to a vertical position, wherein the second probability distribution function is determined in response to second data in form of picture data of the laboratory sample container, and

Fig. 5 depicts highly schematic an apparatus for determining a vertical position of a horizontally extending interface between a first component and a second component.

Fig. 1a depicts a test sample container 4, i.e. a laboratory sample container used for training purposes, containing a first test component 2, e.g. in form of blood serum or an artificial equivalent, and a second test component 3 in form of a gel in layers that are vertically separated from each other, wherein a horizontally extending interface 1 is formed between the first test component 2 and the second test component 3.

The test sample container 4 is depicted in a horizontal orientation due to illustration purposes. Self-evidently, the test sample container 4 and the laboratory sample containers 4 depicted in fig. 1 and the following figures are in fact oriented vertically, i.e. a longitudinal axis of the test container 4 extends in a vertical direction denoted z during the execution of the inventive method.

The vertical position z_pv of the horizontally extending interface 1 is determined based on data generated by sensing a transmittance through the laboratory sample container 4 at different vertical positions z. Reference is insofar made to EP 2 770 317 A1. The vertical position z_pv of the horizontally extending interface 1 is repeatedly determined e.g. 1000 times without changing the measuring setup or test setup, wherein fig. 1b depicts a resulting frequency distribution FD of the repeatedly determined vertical positions z_pv.

Fig. 2a depicts a laboratory sample container 4 containing a centrifuged blood sample comprising a first component 2 in form of blood serum, a second component 4 in form of a gel and a third component 10 in from of cruor in layers that are vertically separated from each other. A horizontally extending interface 1 is formed between the first component 2 and the second component 3, wherein a label 5 is attached to the laboratory sample container 4.

Fig. 2b depicts a first probability distribution function f1, wherein the first probability distribution function f1 assigns a probability P(z) of the presence of the horizontally extending interface 1 depicted in fig. 2a to a vertical position z, wherein the first probability distribution function f1 is determined in response to first data generated by sensing a transmittance through the laboratory sample container 4 at different vertical positions z as e.g. disclosed in EP 2 770 317 A1.

To determine or calculate the first probability distribution function f1, the first data are evaluated, wherein two vertical positions z_pv and z_pv2 are extracted as likely interface positions. The vertical position z_pv corresponds to the true vertical position of the interface 1. The vertical position z_pv2 corresponds to the vertical position of the vertical boarder of the label 5, nevertheless having a reduced probability compared to the position z_pv. The vertical boarder of the label 5 causes a change in transmission, potentially causing a false positive detected interface.

After having computed the two vertical positions z_pv and z_pv2 as likely interface positions, the frequency distribution FD depicted in fig. 1b is weighted with the probability at the corresponding positions z_pv and z_pv2 and is centered at the positions z_pv and z_pv2, respectively.

Fig. 2c depicts a second probability distribution function f2, wherein the second probability distribution function f2 assigns a probability of the presence of the horizontally extending interface 1 depicted in fig. 2a to a vertical position z, wherein the second probability distribution function f2 is determined in response to second data in form of picture data of the laboratory sample container and its content.

The second data are evaluated by means of image processing extracting the vertical position z_pv of the interface 1 and the vertical position z_pv2 of the vertical boarder of the label 5. A frequency distribution describing the variation in extracting the position of the interface 1 and the geometrical properties of the label is weighted with the probability at the corresponding positions z_pv and z_pv2 and centered at the positions z_pv and z_pv2, respectively. Finally, the values are transformed into corresponding probabilities. In the range of the position z_pv the probability of the presence of the horizontally extending interface 1 is positive having a maximum value at the position z_pv. In the range of the position z_pv2 the probability of the presence of the horizontally extending interface 1 is negative having a minimum value at the position z_pv2. Self-evidently, the negative values of the function f2 do not represent negative probabilities. Instead, the negative values are arithmetical values denoting an unlikely interface position.

To finally determine the vertical position z_pv of the horizontally extending interface 1, the first probability distribution function f1 and on the second probability distribution function f2 are combined, e.g. based on Bayes' theorem.

For example using Gauss distribution as probability distribution functions the combined standard deviation $\sigma_{comb}$ is calculated form the individual standard deviations from the individual measurements (first data and second data) $\sigma_i$ as

$$\frac{1}{\sigma_{comb}^2} = \sum_{i=1}^{n} \frac{1}{\sigma_i^2}$$

wherein n is the number of used Gauss-distribution or sensors. Therefore also n>2 sensors or distributions form image processing apparatus can be used to decrease the uncertainty of the measurement.

Since the vertical position z_pv of the horizontally extending interface 1 is determined based on two different probability distribution functions f1 and f2, wherein the probability distribution functions f1 and f2 are based on different sensors, a performant sensor fusion can be achieved providing a more reliable interface detection compared to a solution using only one kind of sensor, i.e. transmission based sensor or a camera based sensor.

In the embodiment depicted in Figures 3a/b/c an additional barcode label 6 is attached to the laboratory sample container 4. The first probability distribution function f1 is computed as mentioned above. The barcode label 6 is detected by means of image processing, wherein the second probability distribution function f2 denotes unlikely probabilities in the range of the barcode label 6.

For instance, a normalized Gauss-distribution with negative values is placed with the center in the center of the bar-code and a 3 $\sigma$ width with the width of the barcode. The negative Gauss distribution can be normalized to values between 0.1 and 0.8 in particular 0.3 or 0.5. This value can be deduced form the generated model, i.e. depending on how often the label edge coincides with the serum-separator/air interface. Alternatively a constant negative probability with a value between 0 and 1 in particular 0,5, 0,75 or 0,9 can be assigned along the barcode area.

In the embodiment depicted in Figures 4a/b/c, the second probability distribution function f2 is generated additionally based on color information in the second date. As depicted, if no color change occurs, the probability of the occurrence of an interface is reduced.

In addition different probabilities can be assigned to different color changes. For instance for a color change between air (transparent) and serum (e.g. light yellow) a positive probability distribution according to the resolution of the measurement principle, e.g. camera resolution, can be assigned to the interface. Also between serum and separation medium (almost white or blue or whatever color the separation medium has) a positive probability distribution can be assigned centered around the color change. The color of the separation medium can be identified by image processing of the whole tube by identifying the type of the tube according to one or several or all of the length and width of the tube and the form and color of the cap as well as the color of the liquid inside the tube. On the other hand, a negative distribution is assigned to a color change between separation medium and blood clot (dark red).

Fig. 5 depicts an apparatus for performing the method as described above.

The apparatus 100 comprises a measurement unit 7a/b, 8a/b being adapted to generate the first data in form of transmission data, wherein the first data depend on the vertical position z_pv of the horizontally extending interface 1.

The apparatus further comprises a camera 8 being adapted to generate the second data in form of picture data of the laboratory sample container 4 containing the first component 2 and the second component 3.

The apparatus further comprises a processing unit 9 being adapted to execute the inventive method.

## Claims

1. Method for determining a vertical position (z_pv) of a horizontally extending interface (1) between a first component (2) and a second component (3), wherein the first component (2) and the second component (3) are contained in a laboratory sample container (4) in layers that are vertically separated from each other, the method comprising the steps:

    - generating first data, wherein the first data depend on the vertical position (z_pv) of the horizontally extending interface (1),
    - generating second data in form of picture data of the laboratory sample container (4) containing the first component (2) and the second component (3),
    - determining a first probability distribution function (f1) in response to the first data, wherein the first probability distribution function (f1) assigns a probability of the presence of the horizontally extending interface (1) to a vertical position (z),
    - determining a second probability distribution function (f2) in response to the second data, wherein the second probability distribution function (f2) assigns a probability of the presence of the horizontally extending interface (1) to a vertical position (z), and
    - determining the vertical position (z_pv) of the horizontally extending interface (1) depending on the first probability distribution function (f1) and on the second probability distribution function (f2),
    - wherein the first data are generated by sensing a transmittance through the laboratory sample container (4) at different vertical positions (z), and
    - **characterised in that** the step of determining the vertical position (z_pv) of the horizontally extending interface (1) depending on the first probability distribution function (f1) and on the second probability distribution function (f2) is performed by combining the first probability distribution function (f1) and the second probability distribution function (f2) based on:

        - Bayes' theorem, and/or
        - Dempster-Shafer theory, and/or
        - Kalman filtering.

2. Method according to one of the preceding claims, **characterized in that** the method comprises the steps:

    - providing a test sample container (4), wherein a first test component (2) and a second test component (3) are contained in the test laboratory sample container (4) in layers that are vertically separated from each other,
    - determining a vertical position (z_pv) of a horizontally extending interface (1) between the first test component (2) and the second test component (3) a number of times,
    - determining a first frequency distribution (FD) of the determined vertical positions (z_pv), and
    - determining the first probability distribution function (f1) in response to the first data and in response to the first frequency distribution (FD).

3. Method according to one of the preceding claims, **characterized in that** the method comprises the steps:

    - providing a test sample container (4) having a label (5) attached to the test sample container (4),
    - determining geometrical properties of the label (5) a number of times,
    - determining a second frequency distribution of the determined geometrical properties, and
    - determining the second probability distribution function (f2) in response to the second data and in response to the second frequency distribution.

4. Method according to one of the preceding claims, **characterized in that** the method comprises the steps:

    - providing a test sample container (4) having a barcode label (6) attached to the test sample container (4),
- determining geometrical properties of the barcode label (6) a number of times,
- determining a third frequency distribution of the determined geometrical properties, and
- determining the second probability distribution function (f2) in response to the second data and in response to the third frequency distribution.

**5.** Method according to one of the preceding claims, **characterized in that** the method comprises the steps:

- generating the second data comprising color information,
- wherein the second probability distribution function (f2) is determined in response to the color information.

**6.** Method according to one of the preceding claims, **characterized in that** the method comprises the steps:

- pipetting the first component (2) and/or the second component (3) in response to the determined vertical position (z_pv) of the horizontally extending interface (1).

**7.** Apparatus (100) for determining a vertical position (z_pv) of a horizontally extending interface (1) between a first component (2) and a second component (3), wherein the apparatus (100) is adapted to perform a method according to one of the preceding claims, wherein the first component (2) and the second component (3) are contained in a laboratory sample container (4) in layers that are vertically separated from each other, the apparatus (100) comprising:

- a measurement unit (7a/b, 8a/b) being adapted to generate first data, wherein the first data depend on the vertical position (z_pv) of the horizontally extending interface (1), wherein the measurement unit (7a/b, 8a/b) is adapted to generate the first data by sensing a transmittance through the laboratory sample container (4) at different vertical positions (z),
- a camera (8) being adapted to generate second data in form of picture data of the laboratory sample container (4) containing the first component (2) and the second component (3), and
- a processing unit (9) being adapted to

- determine a first probability distribution function (f1) in response to the first data, wherein the first probability distribution function (f1) assigns a probability of the presence of the horizontally extending interface (1) to a vertical position (z),
- determine a second probability distribution function (f2) in response to the second data, wherein the second probability distribution function (f2) assigns a probability of the presence of the horizontally extending interface (1) to a vertical position (z), and
- determine the vertical position (z_pv) of the horizontally extending interface (1) depending on the first probability distribution function (f1) and on the second probability distribution function (f2) by combining the first probability distribution function (f1) and the second probability distribution function (f2) based on:

- Bayes’ theorem, and/or
- Dempster-Shafer theory, and/or
- Kalman filtering.

## Patentansprüche

**1.** Verfahren zum Bestimmen einer vertikalen Position (z_pv) einer horizontal verlaufenden Grenzfläche (1) zwischen einer ersten Komponente (2) und einer zweiten Komponente (3), wobei die erste Komponente (2) und die zweite Komponente (3) in einem Laborprobenbehälter (4) in Schichten, die vertikal voneinander getrennt sind, enthalten sind, wobei das Verfahren die folgenden Schritte umfasst:

- Erzeugen von ersten Daten, wobei die ersten Daten von der vertikalen Position (z_pv) der horizontal verlaufenden Grenzfläche (1) abhängen,
- Erzeugen von zweiten Daten in Form von Bilddaten des Laborprobenbehälters (4), der die erste Komponente (2) und die zweite Komponente (3) enthält,
- Bestimmen einer ersten Wahrscheinlichkeitsverteilungsfunktion (f1) als Reaktion auf die ersten Daten, wobei die erste Wahrscheinlichkeitsverteilungsfunktion (f1) eine Wahrscheinlichkeit der Gegenwart der horizontal verlaufenden Grenzfläche (1) einer vertikalen Position (z) zuordnet,
- Bestimmen einer zweiten Wahrscheinlichkeitsverteilungsfunktion (f2) als Reaktion auf die zweiten Daten, wobei die zweite Wahrscheinlichkeitsverteilungsfunktion (f2) eine Wahrscheinlichkeit der Gegenwart der horizontal verlaufenden Grenzfläche (1) einer vertikalen Position (z) zuordnet, und
- Bestimmen der vertikalen Position (z_pv) der horizontal verlaufenden Grenzfläche (1) in Abhängigkeit von der ersten Wahrscheinlichkeitsverteilungsfunktion (f1) und der zweiten Wahrscheinlichkeitsverteilungsfunktion (f2),
- wobei die ersten Daten durch Erfassen einer Durchlässigkeit durch den Laborprobenbehälter (4) an verschiedenen vertikalen Positionen (z) erzeugt werden, und
- **dadurch gekennzeichnet, dass** der Schritt des Bestimmens der vertikalen Position (z_pv) der horizontal verlaufenden Grenzfläche (1) in Abhängigkeit von der ersten Wahrscheinlichkeitsverteilungsfunktion (f1) und der zweiten Wahrscheinlichkeitsverteilungsfunktion (f2) mittels Kombinieren der ersten Wahrscheinlichkeitsverteilungsfunktion (f1) und der zweiten Wahrscheinlichkeitsverteilungsfunktion (f2) basierend auf:

- der Bayesschen Regel und/oder
- der Dempster-Shafer-Theorie und/oder
- dem Kalman-Filtern

durchgeführt wird.

**2.** Verfahren nach einem der vorstehenden Ansprüche, **dadurch gekennzeichnet, dass** das Verfahren die folgenden Schritte umfasst:

- Bereitstellen eines Testprobenbehälters (4), wobei eine erste Testkomponente (2) und eine zweite Testkomponente (3) in dem Testlaborprobenbehälter (4) in Schichten, die vertikal voneinander getrennt sind, enthalten sind,
- mehrmaliges Bestimmen einer vertikalen Position (z_pv) einer horizontal verlaufenden Grenzfläche (1) zwischen der ersten Testkomponente (2) und der zweiten Testkomponente (3);
- Bestimmen einer ersten Häufigkeitsverteilung (FD) der bestimmten vertikalen Positionen (z_pv), und
- Bestimmen der ersten Wahrscheinlichkeitsverteilungsfunktion (f1) als Reaktion auf die ersten Daten und als Reaktion auf die erste Häufigkeitsverteilung (FD).

**3.** Verfahren nach einem der vorstehenden Ansprüche, **dadurch gekennzeichnet, dass** das Verfahren die folgenden Schritte umfasst:

- Bereitstellen eines Testprobenbehälters (4) mit einem Etikett (5), das an dem Testprobenbehälter (4) angebracht ist,
- mehrmaliges Bestimmen geometrischer Eigenschaften des Etiketts (5),
- Bestimmen einer zweiten Häufigkeitsverteilung der bestimmten geometrischen Eigenschaften; und
- Bestimmen der zweiten Wahrscheinlichkeitsverteilungsfunktion (f2) als Reaktion auf die zweiten Daten und als Reaktion auf die zweite Häufigkeitsverteilung.

**4.** Verfahren nach einem der vorstehenden Ansprüche, **dadurch gekennzeichnet, dass** das Verfahren die folgenden Schritte umfasst:

- Bereitstellen eines Testprobenbehälters (4) mit einem Barcode-Etikett (6), das an dem Testprobenbehälter (4) angebracht ist,
- mehrmaliges Bestimmen geometrischer Eigenschaften des Barcode-Etiketts (6),
- Bestimmen einer dritten Häufigkeitsverteilung der bestimmten geometrischen Eigenschaften; und
- Bestimmen der zweiten Wahrscheinlichkeitsverteilungsfunktion (f2) als Reaktion auf die zweiten Daten und als Reaktion auf die dritte Häufigkeitsverteilung.

**5.** Verfahren nach einem der vorstehenden Ansprüche, **dadurch gekennzeichnet, dass** das Verfahren die folgenden Schritte umfasst:

- Erzeugen der zweiten Daten, die Farbinformationen umfassen,
- wobei die zweite Wahrscheinlichkeitsverteilungsfunktion (f2) als Reaktion auf die Farbinformationen bestimmt wird.

**6.** Verfahren nach einem der vorstehenden Ansprüche, **dadurch gekennzeichnet, dass** das Verfahren die folgenden Schritte umfasst:

- Pipettieren der ersten Komponente (2) und/oder der zweiten Komponente (3) als Reaktion auf die bestimmte vertikale Position (z_pv) der horizontal verlaufenden Grenzfläche (1).

**7.** Vorrichtung (100) zum Bestimmen einer vertikalen Position (z_pv) einer horizontal verlaufenden Grenzfläche (1) zwischen einer ersten Komponente (2) und einer zweiten Komponente (3), wobei die Vorrichtung (100) zum Ausführen eines Verfahrens nach einem der vorstehenden Ansprüche ausgelegt ist, wobei die erste Komponente (2) und die zweite Komponente (3) in Schichten, die vertikal voneinander getrennt sind, in einem Laborprobenbehälter (4) enthalten sind, wobei die Vorrichtung (100) Folgendes umfasst:

- eine Messeinheit (7a/b, 8a/b), die zum Erzeugen von ersten Daten ausgelegt ist, wobei die ersten Daten von der vertikalen Position (z_pv) der horizontal verlaufenden Grenzfläche (1) abhängen, wobei die Messeinheit (7a/b, 8a/b) zum Erzeugen der ersten Daten durch Erfassen einer Durchlässigkeit durch den Laborprobenbehälter (4) an verschiedenen vertikalen Positionen (z) ausgelegt ist,
- eine Kamera (8), die zum Erzeugen von zweiten Daten in Form von Bilddaten des Laborprobenbehälters (4), der die erste Komponente (2) und die zweite Komponente (3) enthält, ausgelegt ist, und
- eine Verarbeitungseinheit (9), die ausgelegt ist zum

- Bestimmen einer ersten Wahrscheinlichkeitsverteilungsfunktion (f1) als Reaktion auf die ersten Daten, wobei die erste Wahrscheinlichkeitsverteilungsfunktion (f1) eine Wahrscheinlichkeit der Gegenwart der horizontal verlaufenden Grenzfläche (1) einer vertikalen Position (z) zuordnet,

- Bestimmen einer zweiten Wahrscheinlichkeitsverteilungsfunktion (f2) als Reaktion auf die zweiten Daten, wobei die zweite Wahrscheinlichkeitsverteilungsfunktion (f2) eine Wahrscheinlichkeit der Gegenwart der horizontal verlaufenden Grenzfläche (1) einer vertikalen Position (z) zuordnet, und
- Bestimmen der vertikalen Position (z_pv) der horizontal verlaufenden Grenzfläche (1) in Abhängigkeit von der ersten Wahrscheinlichkeitsverteilungsfunktion (f1) und der zweiten Wahrscheinlichkeitsverteilungsfunktion (f2) durch Kombinieren der ersten Wahrscheinlichkeitsverteilungsfunktion (f1) und der zweiten Wahrscheinlichkeitsverteilungsfunktion (f2) basierend auf:

- der Bayesschen Regel und/oder
- der Dempster-Shafer-Theorie und/oder
- dem Kalman-Filtern.

## Revendications

**1.** Procédé permettant de déterminer une position verticale (z_pv) d'une interface (1) s'étendant horizontalement entre un premier composant (2) et un second composant (3), dans lequel le premier composant (2) et le second composant (3) sont contenus dans un récipient d'échantillon de laboratoire (4) en couches qui sont séparées verticalement les unes des autres, le procédé comprenant les étapes :

- génération de premières données, les premières données dépendant de la position verticale (z_pv) de l'interface (1) s'étendant horizontalement,
- génération de secondes données sous la forme de données d'images du récipient d'échantillon de laboratoire (4) contenant le premier composant (2) et le second composant (3),
- détermination d'une première fonction de distribution de probabilités (f1) en réponse aux premières données, la première fonction de distribution de probabilités (f1) attribuant une probabilité de la présence de l'interface (1) s'étendant horizontalement à une position verticale (z),
- détermination d'une seconde fonction de distribution de probabilités (f2) en réponse aux secondes données, la seconde fonction de distribution de probabilités (f2) attribuant une probabilité de la présence de l'interface (1) s'étendant horizontalement à une position verticale (z), et
- détermination de la position verticale (z_pv) de l'interface (1) s'étendant horizontalement en fonction de la première fonction de distribution de probabilités (f1) et de la seconde fonction de distribution de probabilités (f2),
- dans lequel les premières données sont générées par détection d'un facteur de transmission à travers le récipient d'échantillon de laboratoire (4) au niveau de différentes positions verticales (z), et
- **caractérisé en ce que** l'étape de détermination de la position verticale (z_pv) de l'interface (1) s'étendant horizontalement en fonction de la première fonction de distribution de probabilités (f1) et de la seconde fonction de distribution de probabilités (f2) est réalisée par combinaison de la première fonction de distribution de probabilités (f1) et de la seconde fonction de distribution de probabilités (f2) sur la base :

- du théorème de Bayes, et/ou
- de la théorie de Dempster-Shafer, et/ou
- du filtrage de Kalman.

**2.** Procédé selon l'une des revendications précédentes, **caractérisé en ce que** le procédé comprend les étapes :

- fourniture d'un récipient d'échantillon de test (4), un premier composant de test (2) et un second composant de test (3) étant contenus dans le récipient d'échantillon de laboratoire de test (4) en couches qui sont séparées verticalement les unes des autres,
- détermination d'une position verticale (z_pv) d'une interface (1) s'étendant horizontalement entre le premier composant de test (2) et le second composant de test (3) un certain nombre de fois,
- détermination d'une première distribution de fréquences (FD) des positions verticales (z_pv) déterminées, et
- détermination de la première fonction de distribution de probabilités (f1) en réponse aux premières données et en réponse à la première distribution de fréquences (FD).

**3.** Procédé selon l'une des revendications précédentes, **caractérisé en ce que** le procédé comprend les étapes :

- fourniture d'un récipient d'échantillon de test (4) ayant une étiquette (5) fixée au récipient d'échantillon de test (4),
- détermination des propriétés géométriques de l'étiquette (5) un certain nombre de fois,
- détermination d'une deuxième distribution de fréquences des propriétés géométriques déterminées, et
- détermination de la seconde fonction de distribution de probabilités (f2) en réponse aux secondes données et en réponse à la deuxième distribution de fréquences.

**4.** Procédé selon l'une des revendications précédentes, **caractérisé en ce que** le procédé comprend les étapes :

- fourniture d'un récipient d'échantillon de test (4) ayant une étiquette code-barres (6) fixée au récipient d'échantillon de test (4),
- détermination des propriétés géométriques de l'étiquette code-barres (6) un certain nombre de fois,
- détermination d'une troisième distribution de fréquences des propriétés géométriques déterminées, et
- détermination de la seconde fonction de distribution de probabilités (f2) en réponse aux secondes données et en réponse à la troisième distribution de fréquences.

**5.** Procédé selon l'une des revendications précédentes, **caractérisé en ce que** le procédé comprend les étapes :

- génération des secondes données comprenant des informations de couleur,
- dans lequel la seconde fonction de distribution de probabilités (f2) est déterminée en réponse aux informations de couleur.

**6.** Procédé selon l'une des revendications précédentes, **caractérisé en ce que** le procédé comprend les étapes :

- pipetage du premier composant (2) et/ou du second composant (3) en réponse à la position verticale (z_pv) déterminée de l'interface (1) s'étendant horizontalement.

**7.** Appareil (100) permettant de déterminer une position verticale (z_pv) d'une interface (1) s'étendant horizontalement entre un premier composant (2) et un second composant (3), dans lequel l'appareil (100) est configuré pour réaliser un procédé selon l'une des revendications précédentes, dans lequel le premier composant (2) et le second composant (3) sont contenus dans un récipient d'échantillon de laboratoire (4) en couches qui sont séparées verticalement les unes des autres, l'appareil (100) comprenant :

- une unité de mesure (7a/b, 8a/b) qui est configurée pour générer des premières données, les premières données dépendant de la position verticale (z_pv) de l'interface (1) s'étendant horizontalement, l'unité de mesure (7a/b, 8a/b) étant configurée pour générer les premières données par détection d'un facteur de transmission à travers le récipient d'échantillon de laboratoire (4) au niveau de différentes positions verticales (z),
- une caméra (8) qui est configurée pour générer des secondes données sous la forme de données d'images du récipient d'échantillon de laboratoire (4) contenant le premier composant (2) et le second composant (3), et
- une unité de traitement (9) qui est configurée pour

  - déterminer une première fonction de distribution de probabilités (f1) en réponse aux premières données, la première fonction de distribution de probabilités (f1) attribuant une probabilité de la présence de l'interface (1) s'étendant horizontalement à une position verticale (z),

- déterminer une seconde fonction de distribution de probabilités (f2) en réponse aux secondes données, la seconde fonction de distribution de probabilités (f2) attribuant une probabilité de la présence de l'interface (1) s'étendant horizontalement à une position verticale (z), et
- déterminer la position verticale (z_pv) de l'interface (1) s'étendant horizontalement en fonction de la première fonction de distribution de probabilités (f1) et de la seconde fonction de distribution de probabilités (f2) par combinaison de la première fonction de distribution de probabilités (f1) et de la seconde fonction de distribution de probabilités (f2) sur la base :

  - du théorème de Bayes, et/ou
  - de la théorie de Dempster-Shafer, et/ou
  - du filtrage de Kalman.

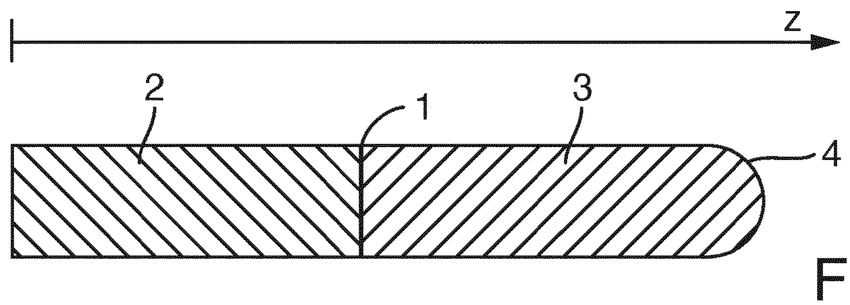


Fig. 1a

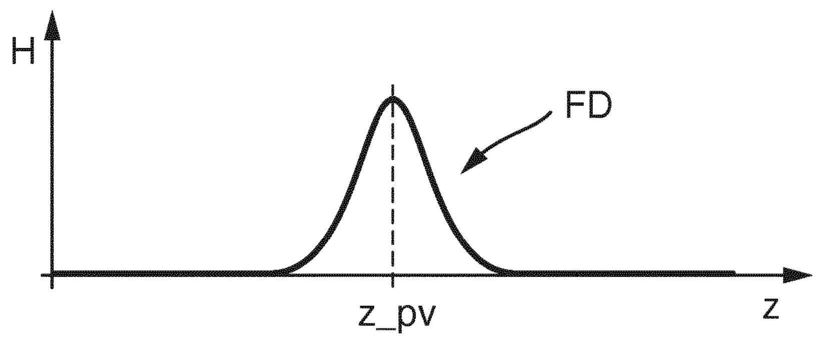


Fig. 1b

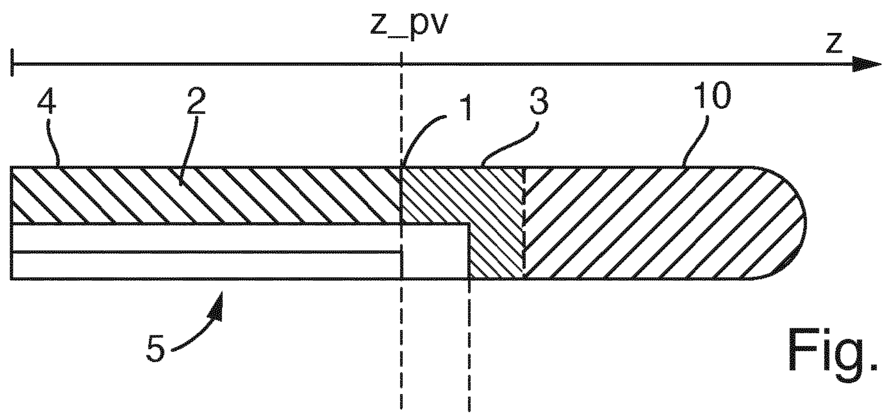


Fig. 2a

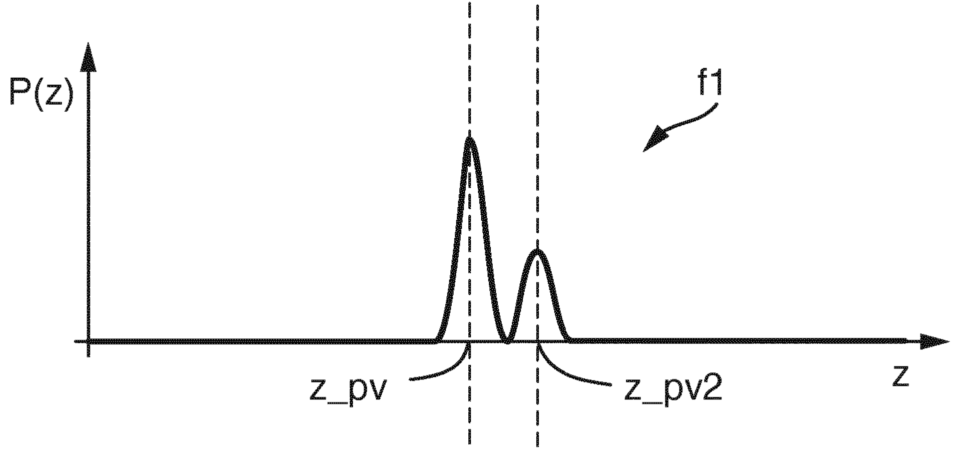


Fig. 2b

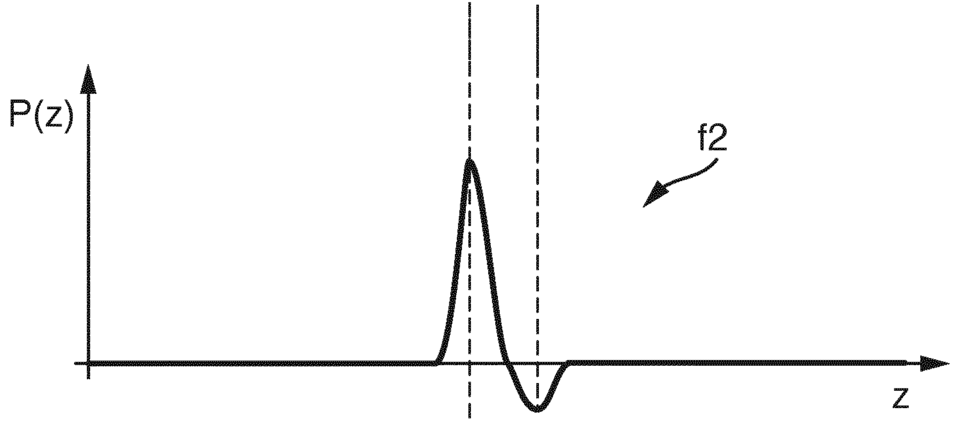


Fig. 2c

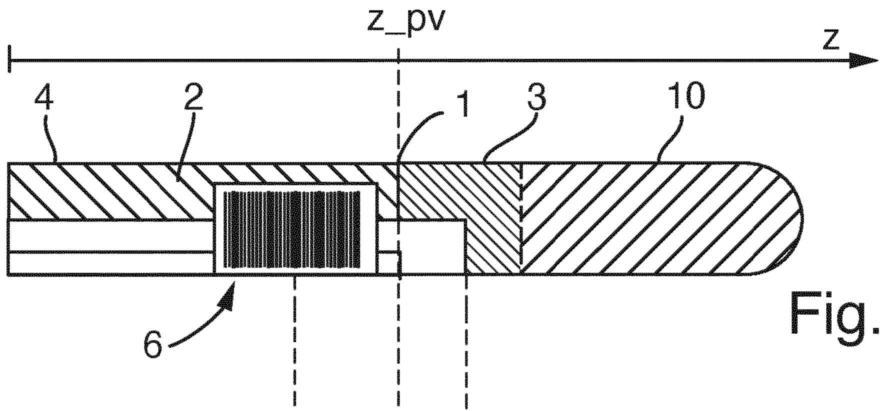


Fig. 3a

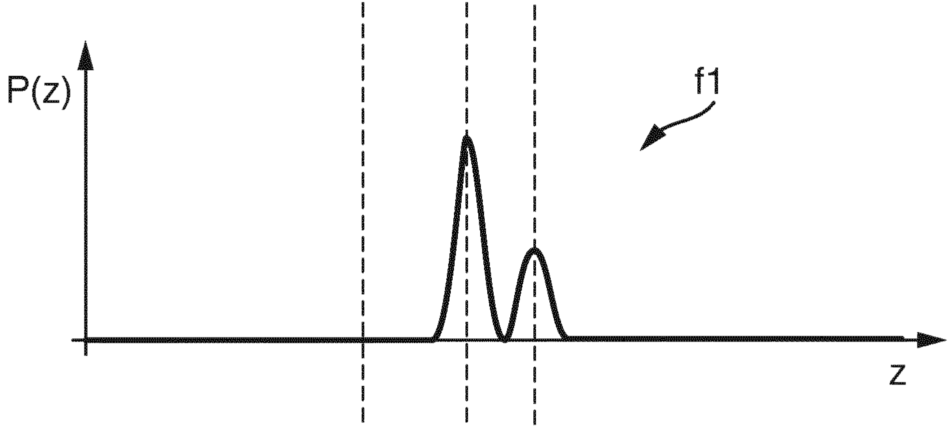


Fig. 3b

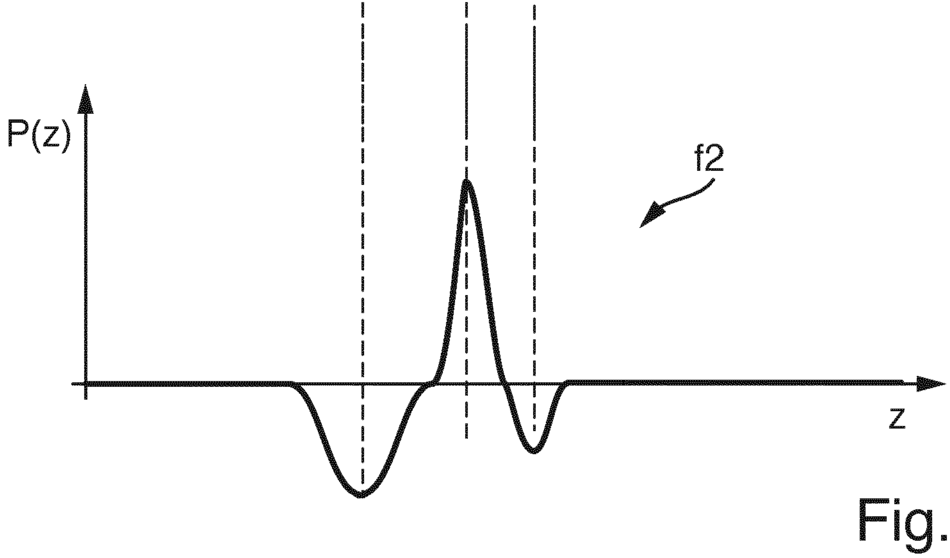


Fig. 3c

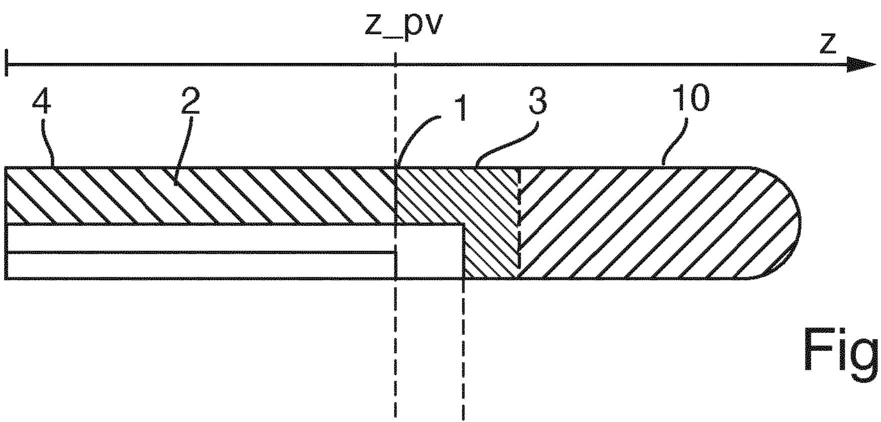


Fig. 4a

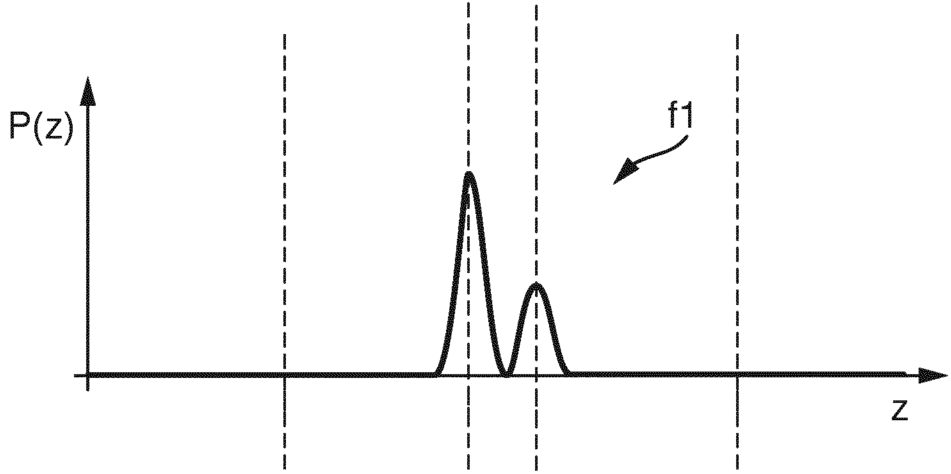


Fig. 4b

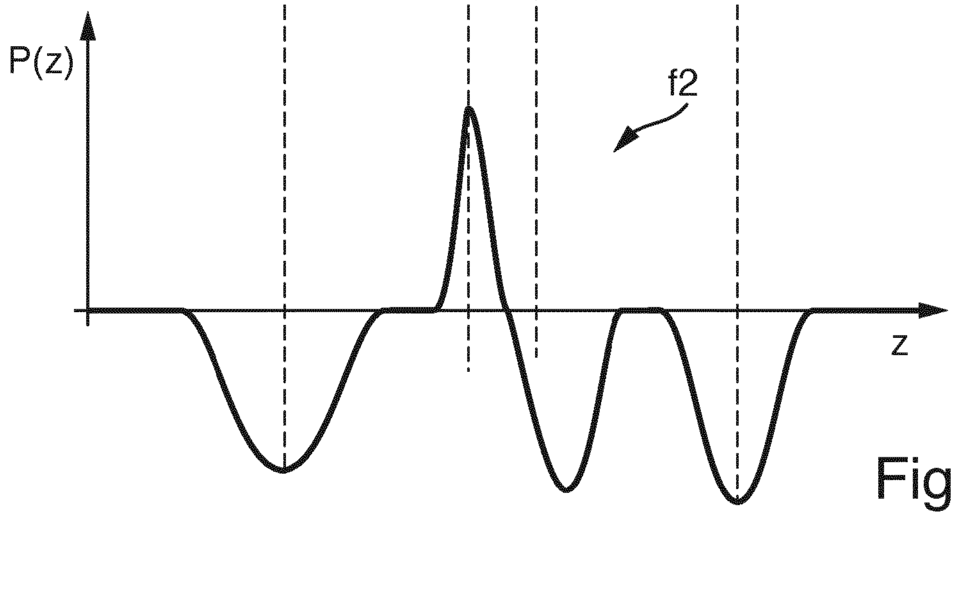


Fig. 4c

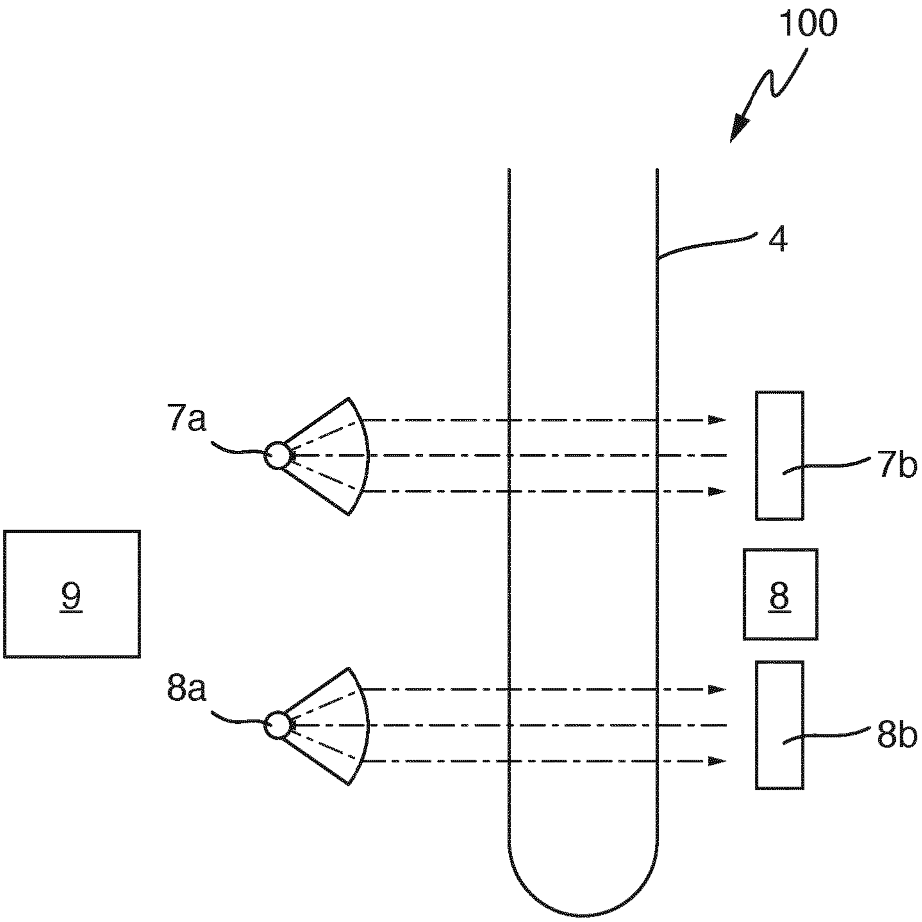


Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2770317 A1
- WO 2019018313 A1
- EP 3467452 A1
- JP 2014006094 A